# NEW EUROPEAN PATENT SPECIFICATION

(11) **EP 0 275 249 B2**
(45) Date of publication and mention of the opposition decision: **17.12.1997**
(45) Mention of the grant of the patent: 13.01.1993
(21) Application number: 85905544.4
(22) Date of filing: 29.10.1985
(51) Int. Cl.: A61K 31/70, C07H 19/167

(54) **CONTINUOUS INTRAVENOUS INFUSION OF ADENOSINE TO HUMAN PATIENTS, A UNIT DOSAGE FORM OF ADENOSINE AND USE OF ADENOSINE FOR THE MANUFACTURE OF MEDICAMENTS**
KONTINUIERLICHE INTRAVENÖSE INFUSION VON ADENOSIN AN MENSCHLICHEN PATIENTEN, EINHEITSDOSIERUNGSFORM VON ADENOSIN UND DESSEN VERWENDUNG BEI DER HERSTELLUNG VON ARZNEIMITTELN
INJECTION INTRAVEINEUSE EN CONTINU D'ADENOSINE A DES PATIENTS HUMAINS, FORME DE DOSAGE UNITAIRE D'ADENOSINE ET EMPLOI D'ADENOSINE POUR LA FABRICATION D'UN MEDICAMENT

(30) Priority: 24.09.1985 US 779516
(43) Date of publication of application: 27.07.1988
(60) Divisional application: 92201323.0
(73) Proprietor: ITEM DEVELOPMENT AB, S-182 71 Stocksund (SE)
(72) Inventor: SOLLEVI, Alf, S-161 55 Bromma (SE)
(74) Representative: Fogelberg, Lennart
(86) International application number: SE8500423
(87) International publication number: WO8701593

(56) References cited:
- EP-A- 0 012 272
- EP-A- 0 054 635
- EP-A- 0 152 944
- DE-A- 3 133 925
- US-A- 3 823 234
- US-A- 3 845 205
- US-A- 4 088 756
- US-A- 4 364 922
- J.Neurosurg. 58: 69-76, 1983
- Anesthesiology 61: 400-405 (1984)
- Scand J Thor Cardiovasc Surg 19: 155-159, 1985
- Thoracic and cardiovascular Surgeon, vol. 30, no. 1, 1982 (new York), T.N. Masters et al., pp. 14-20
- Chest, vol. 83, no. 5, May 1983 (Chicago), N.A. Silverman et al., pp. 787-792
- Acta Physiologica Scandinavica, vol. 120, no. 2, 1984 (Stockholm), A. Sollevi et al., pp. 171-176
- Chemical Abstracts, vol. 33 (1939), abstract no. 2362, Acta Med. Scand., Suppl. 89, 239-44 (1938)
- Chemical Abstracts, vol. 40 (1946), abstract no. 19267, Helv. Physiol. Pharmacol. Acta 3, 621-36 (1945)
- Chemical Abstracts, vol. 53 (1959), abstract no. 12484b, Arch. exptl. Pathol. Pharmakol. 236, 226-9 (1959)
- Chemical Abstracts, vol. 98 (1983), abstract no. 100933y, J. Neurosurg. 1983, 58(1), 69-76
- Chemical Abstracts, vol. 99 (1983), abstract no. 206877u, Acta Physiol. Scand. 1983, 119 (3), 225-30
- Chemical Abstracts, vol. 101 (1984), abstract no. 116749j, Rom RO 82,025
- Surgery, Vol. 78, 1975, G. Frank O. Tyers et al, p. 45
- J. Thorac. Cardiovasc. Surg., Vol. 80, 1980, p. 506
- Circulation Research, Vol. 47, 1980, Robert M. Berne, p. 807
- Arzneimittel-Forschung, 2. Beiheft, 1952, Editio Cantor, S.51 ff, W. Herbrand, K.H. Jaeger
- J. Pharmacol. (Amer.), Vol. 41, 1931. p. 355, A.M. Wedd
- Scand. J. Thorac. Cardiovasc. Surg., Vol. 30 (Suppl.), 1981, pp. 1-28, P. Jynge et al
- Circulation Research, Vol. 36, 1975, p. 481-489, D.J. Heanse :"Hypothermic arrest and Potassium arrest"
- Cardiac Anesthesia Second Edition, Vol. 2, 1987, Joel A. Kaplan, pp.902,903, 927-962

## Description

This invention is concerned with the use of adenosine as an agent for the treatment of human beings. More particularly, this invention is concerned with a solution for inducing cardioplegia, a process for preparation of such solution and the use of adenosine in a process for manufacture of a pharmaceutical preparation for inducement of cardioplegia during cardiovascular surgery.

Adenosine is a naturally occurring nucleoside composed of the purine, adenine, and the sugar, D-ribose. Normal basal plasma levels of adenosine are from about 0.1 to about 0.2 µmol per liter. In addition, it is commonly present in the body in the form of adenosine monophosphate (AMP), adenosine diphosphate (ADP) and adenosine triphosphate (ATP). Adenosine has been reported to have a variety of biological effects, depending on whether the adenosine is endogenous or exogenously administered, including sedative and anti-epileptic effects on the central nervous system and inhibitory effects on respiration, cardio-vascular effects including prolongation of atrio-ventricular conduction time and impulse formation in the sinus node, vasodilation, antiaggregatory effect, decreased release of free fatty acids, anti-secretory effect in the stomach, and anti-diuretic effect.

As a general rule, however, adenosine and its biological effects have been largely of physiological interest. To the extent adenosine was of interest as a pharmaceutical product, that interest has centered primarily on its phosphate derivative, which now is known to be rapidly metabolized to yield adenosine and phosphate in the circulation. See Sollevi et al, Acta. Physiol. Scand. 120:171-6 (1984). However, phosphate may cause undesired side effects. For example, high levels of phosphate my cause arrhythmias secondary to chelation of magnesium and calcium. (See Dedrick, et al., Anesthesiology, 57:3A, 66 (1983).

Furthermore, adenosine is known to produce heart block through blockage of the atrioventricular (A-V) node. As a consequence, methylxanthines such as theophylline have been proposed by Berne, et at., in U.S. Patent No. 4 364 922 for use in preventing heart block caused by adenosine, in particular adenosine released as a consequence of cardiac ischemia or hypoxia.

In addition, it has been proposed to take advantage of adenosine's ability to block atrioventricular conductance by using it to treat tachyarrhythmias. For such use, adenosine is administered as an injectable intravenous bolus containing from about 37.5 micrograms/kg up to about 450 micrograms/kg of adenosine. In such a use, the adenosine has little detectable vasodilating action. Adenosine has a very short plasma half-life, of the order of 10-20 seconds (see, Fredholm and Sollevi,J. Physiol., 313:351-62 (1981)), and thus the concentration of injected adenosine is rapidly reduced to normal serum levels (about 0.15 µmol per liter). The transitory presence of the injected adenosine precludes all but the most transitory vasodilation.

Accordingly, for adenosine to be of practical value for use as a vasodilator, it must be administered continuously to maintain plasma levels sufficiently high to achieve vasodilation. The problem, however, is that such continuous administration could lead to undesired side effects, such as the above noted heart blockage.

It also should be noted that compounds commonly used as vasodilators, such as sodium nitroprusside, nitroglycerine. isoflurane, hydralazine, prazosin and the like, have various side effects. For example, sodium nitroprusside has the drawbacks of tachyphylaxis and rebound hypertension, apparently caused by autogenous generation of angiotensin to counteract the hypotensive effect of nitroprusside. As a consequence, the dosage of nitroprusside must be progressively increased with continued use to overcome the hypertensive effect of angiotensin, and there is a risk of rebound due to the presence of residual excess angiotensin. Nitroglycerine and prazosin suffer from the drawbacks of slow onset and unpredictable action. Isoflurane and sodium nitroprusside both have a tendency to reduce cardiac blood flow, while nitroprusside, hydralazine and prazosin increase heart rate.

Silverman, N. A. and Feinberg, H., in Chest, vol. 83, no. 1, 1983, pages 787-792, report the use of adenosine as a substrate for restitution of high energy phosphates (ATP) after a period of conventional potassium cardioplegia. The report indicates beneficial effects for this use of adenosine during reperfusion of the heart.

The inventor has dicovered that adenosine can be administered to human patients under conditions such that significant vasodilation is achieved without the occurrence of significant heart blockage. He further discovered that adenosine has a unique, and heretofore unappreciated, activity profile in humans which differs significantly from the profiles of heretofore commonly used vasodilators. As a consequence of this discovery, it has been discovered that adenosine may be employed for the treatment of a variety of conditions by continuous intravenous infusion techniques. In particular, and as will be illustrated in greater detail below, adenosine has been found to have the following characteristics:
1. It has selective vasodilation activity, in that its effect is limited to a cardiac after-load effect. That is, its activity is limited to dilation of arteries and it has little or no effect on cardiac pre-load. i.e. as a dilator of vein.
2. Although adenosine has significant action in blocking atrio-ventricular (A-V) conductance by bolus injection, it can be administered by continuous infusion and have significant useful vasodilation action at dosages below those at which it has significant A-V activity.
3. Adenosine has significant hypotensive activity without the occurrence of significant tachyphylaxis, apparently because adenosine blocks the renin-angiotensin system of the kidney, thus preventing hypertension due to the formation of angiotensin in response to hypotension.
4. Adenosine's effect is readily controlled because it is active at relatively small doses and because of its short plasma half-life (10-20 seconds). In addition, its activity quickly ceases when adenosine administration is terminated.
5. Adenosine is capable of significantly increasing cardiac output without significantly increasing cardiac work.
6. Adenosine, in the amounts used in accordance with the invention, is essentially non-toxic. It is rapidly taken up by the body to form ATP, and upon degradation its metabolites are present at or below levels normally resulting from physical exercise.

Adenosine can be administered to the patient in any pharmaceutically acceptable form suitable for use in continuous, intravenous infusion. A preferred form is an aqueous solution of adenosine, and more preferably adenosine in isotonic saline. The concentration of adenosine is not narrowly critical, although concentrations of at least 5 millimol (or about 1.5 milligrams per milliliter) of solution are desired to avoid the need for excessive infusion rates to achieve desired serum levels. The concentration can be up to the solubility limit of adenosine (about 20 millimols per liter or 5.5 to 6 milligrams per milliliter) if desired.

When used for continous infusion, the unit dosage form typically has a volume of at least 250 milliliters, and preferably in the range of 250 to 500 milliliters, to provide an adequate supply of adenosine. Consequently, the unit dosage form generally will contain from about 0.4 to about 3 grams of adenosine.

Of course, the adenosine solution should be sterile and free from fungi and bacteria. Such solutions have been found to be stable at room temperature for at least two years.

Such solutions are prepared by mixing adenosine with the aqueous carrier, e.g. water or an isotonic solution, and other desired ingredients, to achieve a solution having the desired concentration, and thereafter sterilizing the solution.

Continuous infusion can be achieved by any technique known to the art. Because adenosine has such a short plasma half-life and it is active at relatively low concentrations, it is desired that the method be one which minimizes or avoids fluctuations of serum adenosine levels. Accordingly use of high precision roller pumps is preferred.

### Coronary vasodilation

It has been further found that when adenosine is administered by infusion at rates which do not induce significant hypotension, it has clinically useful regional effects in unanesthetized and anesthetized patients.

For example, adenosine at dosages of the order of 10 to 15 per cent of hypotensive levels (e.g. 0.02 to 0.05 mg per kg per minute) can be a useful adjunct to coronary by-pass surgery, apparently due to a preferential coronary vasodilation. It has been reported that coronary artery grafts occlude more frequently during the postoperative period when low graft-flow values are obtained during surgery. See Groudin et al, Circulation, 42: Suppl 3: 106-111 (1970). It has been found that low doses of adenosine administered postoperatively increase graft blood flow without significant effect on atrio-ventricular conductance. The administration of low doses of adenosine for this purpose can be carried out for as long as is necessary to afford appropriate graft flows and reduce risk of occlusion, but ordinarily the period need not exceed 48 hours following surgery.

In a study designed to investigate the use of adenosine to inhibit occlusion of coronary grafts, nine patients (age 45-65, all taking beta-blockers) were studied during coronary artery surgery. After premedication, morphine (10-15 mg) and scopolamine (0.4-0.6 mg), anesthesia was induced by fentanyl (30 mcg/kg b.w.). Pancuronium (0.1 mg/kg b.w.) was given to facilitate endotracheal intubation. Anesthesia was maintained with fentanyl 0.5 mg/hour, N₂O (50%) in oxygen and droperidol (0.1 mg/kg b.w.). During bypass thiomebumal (5 mg/kg b.w.) was given. Nitrous oxide was not used after bypass. Extracorporeal circulation (ECC) was performed with a roller pump and a Shiley buddle oxygenator primed with crystalloid solution. ECG (modified V₅), an arterial line and a Swan-Ganz Catheter were used for monitoring and for hemodynamic measurements. Blood flow in bypass grafts (n=15, internal mammary and venous grafts), was measured with appropriate sizes square wave electromagnetic flow probes (Nycotron 732). The study was performed 20-30 minutes after the termination of ECC. After a control period (5 min), adenosine (5.3 mg/ml clinical solution) was continuously infused in a central vein in order to induce approximately 10 % reduction of mean arterial blood pressure (about 30 to 50 µg per kg per min). Graft flow was continuously measured before and during a 10 or 30 minute infusion of adenosine and finally during the following 5 minute control period. Data are expressed as mean ± SEM and differences were tested with Student's paired t-test against the preceding period.

The results of this study are summarized in Table I.

**Table I**

| | Control Before | Adenosine | Control After |
|---|---|---|---|
| Mean Arterial Pressure (mmHg) | 84±3 | 74±3 p<0.01 | 85±3 p<0.01 |
| Heart Rate (beats/min) | 82±5 | 82±15 | 81±6 |
| Cardiac Output | 4.8±0.4 | 5.6±0.3 p<0.05 | 5.3±0.3 n.s |
| Pulmonary Artery Pressure (mean) (mmHg) | 16.7±1.2 | 18.8±1.2 | 19.9±1.0 |
| RAP(mean) (mmHg) | 4.7±0.5 | 5.3±0.4 | 5.8±0.7 |
| Stroke Index (ml/m²) | 35.6±2.6 | 38.8±2.0 | 39.6±2.5 |
| Left Ventricular Stroke Index (Joule/n²) | 0.44±0.03 | 0.41±0.03 | 0.49±0.04 |
| Graft flow ml/min (n=15) | 40 + 5 | 77±7 p<0.001 | 39±5 p<0.001 |

As is evident from Table I, adenosine in a dose of 40 ± 4 µg/kg/minute, a level which reduced mean arterial pressure 12 %, increase cardiac output 12 %, and doubled graft flow. At the same time, heart rate, mean pulmonary artery pressure, central venous pressure, stroke index and left ventricular stroke work index remained essentially unchanged. Graft flow rate was restored to its original value on termination of adenosine. No arrhythmias were observed.

This demonstrates that i.v. adenosine at low rates (30-50 µg per kg per min) induces a marked and reproducible increase in graft flow without increased myocardial work, apparently due to preferential vasodilatory effect of adenosine in the coronary vasculature.

### Platelet protection during cardiopulmonary bypass

The following chapter corresponds largely to the disclosure of Scand J Thor Cardiovasc Surg 19:155-159 (1985).

Continuous infusion of adenosine also has been found of use in protecting platelets during cardiopulmonary bypass. For such use, it is desired to maintain the adenosine dosage below that affording significant vasodilation, and a rate of about 100 µg/kg/min has been found effective. In contrast, prostacyclin, a prostaglandin used to inhibit platelet aggregation, is associated with severe systemic vasodilation and hypotension during coronary bypass surgery.

Twenty-five patients scheduled for coronary artery bypass surgery were randomly assigned to two groups, one with adenosine infusion (n=13) and the other with placebo infusion (n=12).

Routine tests of coagulation status were normal in all patients, and none was taking drugs known to affect platelet function. Intravenous anesthesia was used, either high-dose fentanyl (100-150 µg/kg) or balanced anesthesia (thiopental, fentanyl diazepam and N₂O/O₂).

During cardiopulmonary bypass (CPB), mean arterial blood pressure (MABP) above 70 mmHg was treated with the vasodilator sodium nitroprusside (SNP), except in the final phase or rewarming. CPB was performed with SARN roller pump and a Shiley oxygenator (100A) primed with 2000 ml crystalloid solution (75 mg heparin). The perfusion rate was kept at approximately 1.8 ml/m² body surface. Moderate hypothermia (25 °C) was induced. Cardioplegia was obtained with Ringer's solution (with added potassium up to 20 mM/1).

Heparin (3 mg/kg) was administered as a bolus injection before cannulation. The heparin effect was controlled by measurements of activated clotting time. (Hemocron^{R} Int.Technidyne Corp, USA). This time (ACT) was > 400 seconds in all patients during CPB. At the termination of CPB, the heparin effect was antagonized with protamine (c. 1.3 mg/mg heparin). ACT was checked 10-20 min after the protamine injection. ACT values 120 sec were considered satisfactory.

Platelet count (Linson 431 A cell counter) and hematocrit were determined in arterial samples before anesthesia, after thoracotomy, during CPB at 10, 20, 40, 60, 80 and 100 min, 30 min after CPB and on the postoperative day. Platelet counts were expressed in percentage of preanesthesia levels and were corrected for hemodilution. MABP was monitored continuously via a catheter introduced in the radial artery. All patients received hypertonic mannitol (1-1.5 g/kg) during CPB and urine production was calculated as ml/min of CPB (table II).

Peroperative blood loss and blood transfusions could not be compared between the groups, due to the smallness of the series and the involvement of many surgeons in the study. Postoperative bleeding was measured as the blood loss from the tube drainage from the end of operation until the postoperative morning. One patient in the adenosine group was excluded because of reoperation for surgical reasons (for massive bleeding due to suture insufficiency in a graft anastomosis) within 6 hours after CPB.

Adenosine (5.3 mg/ml, clinical solution ) was infused at a rate of 100 µg/kg/min into the superior vena cava throughout CPB. The adenosine dose was based on five pilot cases in which a vasodilation dose-response was observed. The highest infusion rate that did not induce systemic vasodilation was chosen for this study. In six cases the plasma adenosine levels were determined by high performance liquid chromatography (HPLC) (Fredholm and Sollevi, J. Physiol. (London), 313: 351-67 (1981) in arterial and in venous (venous lines to the oxygenator) blood. The adenosine metabolites inosine, hypoxanthine and uric acid were also determined by HPLC. The samples were collected as previously described (Sollevi, Acta. Physiol. Scand., 121: 165-72 (1984) at the intervals of 10, 20, 40 and 80 minutes during CPB and 20 minutes after CPB.

Results are summarized in Tables II and III, below in which data are presented as means ± SEM. Statistical significance (controls v, adenosine group) was determined with Student's t-test for unpaired data. For significance within the groups the Wilcoxon Rank Sum test was used. p < 0.05 was regarded as significant.

**Table II**

| Patient data | Adenosine | mg per kg per min |
|---|---|---|
| | 0 | 0.1 |
| Age, years | 57 (range 47-66) | 57 (range 42-74) |
| Males/females | 11/1 | 12/1 |
| CPB-time (min) | 95±10 | 120±10 |
| Preoperative platelet counts(x 10⁹ cells/l) | 162 (range 107-235) | 158 (range 106-251) |
| Peroperative urine production (ml/min CPB) | 9.7 (2.1-20) | 4.1 (1.1-9.5) |
| Postoperative blood loss (ml) | 630±60 | 640±80 |

**Table III**

| Arterial and venous concentrations (µM) of adenosine and its metabolites (n=6), before, during and after adenosine infusion (0.1 mg x kg-1 x min-1). | | | | | | |
|---|---|---|---|---|---|---|
| | pre-CBP | CPB | | | | CPB 20 post |
| | | 10' | 20' | 40' | 80' | |
| | | | | | | |

| Vein | | | | | | |
|---|---|---|---|---|---|---|
| Adenosine | 0.3±0.2 | 3.7±1.3* | 5.7±2.1* | 4.5±1.1* | 3.0±1.0* | 0.4±0.1 |
| Inosine | 0.2±0.1 | 0.9±0.3* | 2.4±1.2* | 1.6±0.5* | 1.6±0.4* | 0.4±0.1 |

| Artery | | | | | | |
|---|---|---|---|---|---|---|
| Adenosine | 0.3±0.1 | 0.7±0.3 | 0.8±0.4* | 0.6±0.3 | 0.4±0.2 | 0.3±0.1 |
| Inosine | 0.2±0.1 | 1.3±0.7* | 2.5±1.0* | 1.2±0.3* | 1.4±0.4* | 0.4±0.1 |
| Hypoxanthine | 3.2±0.8 | 5.3±1.2 | 7.7±1.4* | 5.3±1.0 | 5.0±0.9 | 4.2±0.9 |
| Uric Acid | 250±30 | 260±32 | 269±35 | 250±32 | 249±30 | 260±34 |

| | | | | | | |
|---|---|---|---|---|---|---|
| *Significantly different from pre-CPB value. | | | | | | |

**Table IV**

| | Platelet count; % of Awake count | |
|---|---|---|
| Time of count | Control | Adenosine |
| Awake | 100 | 100 |
| Pre CPB | 95 | 97 |

| During CPB (Duration 100 minutes) | | |
|---|---|---|
| 10 minutes | 80 | 96 |
| 20 minutes | 75 | 87 |
| 40 minutes | 65 | 85 |
| 60 minutes | 80 | 91 |
| 80 minutes | 78 | 100 |
| 100 minutes | 77 | 97 |

| Post CPB, time after infusion of CPB | | |
|---|---|---|
| 30 minutes | 70 | 88 |
| 24 hours | 60 | 60 |

As shown in Table IV, platelet count was similar in the two groups before anesthesia and was unaltered by anesthesia and thoracotomy. In the control group the platelet count fell rapidly and markedly during the first 40 minutes of CPB and remained significantly reduced during and after CPB. During adenosine infusion the initial platelet reduction was small, and was significant only at 20 and 40 minutes on CPB. From 60 minutes to the end of CPB and 30 minutes after CPB there was significant intergroup difference in platelet counts. On the day after operation the platelet counts were markedly reduced in both groups, with no significant intergroup difference.

As shown in Table III, the arterial and venous adenosine levels were in the normal range of 0.3 µM prior to CPB. The adenosine infusion raised the venous plasma concentration to 2-10 µM, while the arterial levels were approximately doubled during the initial CPB period. Only the first adenosine metabolite, inosine, was consistently elevated during the infusion.

All parameters had returned to control levels within 20 minutes after CPB.

The mean arterial blood pressure (MABP) did not differ significantly between the two groups during CPB. In the placebo group, however, seven patients required sodium nitroprusside infusion (< 5 µg/kg/min) to keep MABP below 70 mmHg. No patient in the adenosine group required vasodilator treatment. After CPB the patients in the adenosine group had slightly lower MABP, but at the end of operation there was no intergroup difference. The urine production during CPB was 250 ml/h in the adenosine group and 500 ml/h in the controls (p<0.01, Table II). Transient elevation of serum creatinine levels (10-20 % above normal range) was found in two patients in the adenosine group and one control patient during two postoperative days. The postoperative blood loss did not differ between the groups.

All patients were extubated within 24 hours after the operation and all recovered normally. There were no clinical signs of neurologic complications and all the patients were discharged from the hospital.

### Addition of adenosine to cardioplegia solution.

Cardioplegia is induced during open heart surgery in order to arrest the heart and to reduce myocardial oxygen consumption during cardiopulmonary bypass. This is at present generally obtained by an ice-cooled solution containing high concentration of potassium (20 mmol/liter, four times the normal serum level) that is infused into the coronary vessels. It is well known that high concentrations of potassium effectively induce asystole, but also cause damage on vascular endothelium. The latter may lead to permanent stenosis of coronary vessels.

The foregoing data has clearly demonstrated in human patients that adenosine is effective as coronary vasodilator and preserves circulating platelets. Adenosine is also known to be incorporated into high energy phosphates (ATP) in various tissues. In addition, it is well known since the early work of Drury and Szent Gyorgyi (J.Physiol (London) 68:213 (1929)) that high concentration of adenosine can produce heart block.

These four effects of adenosine are all useful during the induction of cardioplegia in human patients. First, the vasodilatory effect can counteract the vasoconstrictor effect of potassium and thereby reduce the time required for administration of cardioplegia solution. This will give a more rapid cooling and thereby more rapid asystoli. Secondly, the inhibitory effect of adenosine on platelet activation can prevent platelet aggregation in the coronary circulation during this cooling phase. Third, adenosine can serve as a substrate and be incorporated into myocardial ATP during this condition, when the heart is beating without obtaining adequate oxygen supply. Finally, the well known A-V blocking effect of high concentrations of adenosine can be used for the induction of asystoli. Then, the potassium concentration of the cardioplegia solution can be reduced to a level that does not damage the vascular endothelium.

These four effects of adenosine can all be achieved with a cardioplegia solution containing 0.5-1.5 mg/ml of adenosine, administered into the coronary circulation during the induction of cardioplegia. Ordinarily, when adenosine is administered for this purpose through the aortic root, the cardioplegia solution will be administered at the rate of 50 to 150 ml/min over a period of 10 to 20 minutes.

## Claims

1. A potassium solution for inducing cardioplegia during open heart surgery on a human patient, characterized in that said solution has a potassium concentration substantially less than a usual cardioplegic potassium solution, such as a usual cardioplegic potassium solution having a potassium concentration of 20 mmol/l, and that said solution further comprises adenosine in an amount sufficient to facilitate K⁺-induced cardioplegia.

2. A solution according to claim 1 characterized in that the amount of adenosine is 0.5-1.5 mg/ml.

3. A process for preparing a solution according to claim 1, comprising dissolving a potassium salt to prepare a cardioplegic solution, characterized in preparing said solution with substantially less potassium than usual, such as to a concentration substantially less than 20 mmol/l, and further dissolving an amount of adenosine effective to facilitate K⁺-induced cardioplegia in a human patient.

4. A process according to claim 3 characterized in that the dissolving amount of adenosine is 0.5-1.5 mg/ml.

5. Use of adenosine as a physiologically effective additive to a cardioplegic solution in a process for manufacture of a pharmaceutical preparation for inducement of cardioplegia during open heart surgery.

6. Use as claimed in claim 5 wherein the process comprises dissolving potassium to a concentration substantially less than 20 mmol/l.

7. Use as claimed in claim 5 or 6 whereby 0.5-1.5 mg of adenosine is included per ml of said solution.

## Patentansprüche

1. Kaliumlösung zur Herbeiführung von Kardioplegie während offener Herzoperation bei einem menschlichen Patienten, **dadurch gekennzeichnet**, daß die Lösung eine wesentlich geringere Kaliumkonzentration als eine übliche Kardioplegie-Kaliumlösung, wie eine übliche Kardioplegie-Kaliumlösung mit einer Kaliumkonzentration von 20 mmol/l, hat und daß die Lösung außerdem Adenosin in einer ausreichenden Menge umfaßt, um durch K⁺ herbeigeführte Kardioplegie zu erleichtern.

2. Lösung nach Anspruch 1, **dadurch gekennzeichnet**, daß die Adenosinmenge 0,5 bis 1,5 mg/ml beträgt.

3. Verfahren zur Herstellung einer Lösung nach Anspruch 1 unter Auflösung eines Kaliumsalzes zur Herstellung einer Kardioplegielösung, **dadurch gekennzeichnet**, daß man die Lösung mit wesentlich weniger Kalium als üblich, wie bis zu einer Konzentration wesentlich geringer als 20 mmol/l bereitet und außerdem eine wirksame Adenosinmenge auflöst, um bei einem menschlichen Patienten mit K⁺ herbeigeführte Kardioplegie zu erleichtern.

4. Verfahren nach Anspruch 3, **dadurch gekennzeichnet**, daß die aufgelöste Adenosinmenge 0,5 bis 1,5 mg/ml beträgt.

5. Verwendung von Adenosin als physiologisch wirksames Additiv zu einer Kardioplegie-Lösung in einem Verfahren zur Herstellung eines pharmazeutischen Präparates zur Herbeiführung von Kardioplegie während offener Herzoperation.

6. Verwendung nach Anspruch 5, bei der das Verfahren ein Auflösen von Kalium bis zu einer Konzentration wesentlich geringer als 20 mmol/l umfaßt.

7. Verwendung nach Anspruch 5 oder 6, bei der 0,5 bis 1,5 mg Adenosin je Milliliter der Lösung enthalten sind.

## Revendications

1. Solution de potassium pour induire une cardioplégie au cours d'une opération de chirurgie à coeur ouvert pratiquée sur un patient humain, caractérisée en ce que ladite solution a une concentration en potassium sensiblement inférieure à celle d'une solution de potassium usuelle pour cardioplégie ayant une concentration en potassium de 20 mmoles/l, et en ce que ladite solution comprend en outre de l'adénosine présente en une quantité suffisante pour faciliter une cardioplégie induite par K⁺.

2. Solution selon la revendication 1, caractérisée en ce que la quantité d'adénosine est de 0,5 à 1,5 mg/ml.

3. Procédé pour préparer une solution selon la revendication 1, comprenant la dissolution d'un sel de potassium pour préparer une solution cardioplégique, caractérisé en ce qu'on prépare ladite solution avec sensiblement moins de potassium que la quantité usuelle, par exemple jusqu'à une concentration sensiblement inférieur à 20 mmoles/l, et l'on dissout en outre une quantité d'adénosine efficace pour faciliter une cardioplégie induite par K⁺ chez un patient humain.

4. Procédé selon la revendication 3, caractérisé en ce que la quantité d'adénosine dissoute est de 0,5 à 1,5 mg/ml.

5. Utilisation d'adénosine comme additif physiologiquement efficace à une solution cardioplégique dans un procédé de fabrication d'une préparation pharmaceutique destinée à induire une cardioplégie au cours d'une opération de chirurgie à coeur ouvert.

6. Utilisation telle que revendiquée dans la revendication 5, dans laquelle le procédé comprend la dissolution de potassium jusqu'à une concentration sensiblement inférieure à 20 mmoles/l.

7. Utilisation telle que revendiquée à la revendication 5 ou 6, et selon laquelle une quantité de 0,5 à 1,5 mg d'adénosine est incluse par ml de ladite solution.
